# EUROPEAN PATENT SPECIFICATION

(11) **EP 3 576 789 B1**
(45) Date of publication and mention of the grant of the patent: **11.12.2024**
(21) Application number: 18747516.5
(22) Date of filing: 01.02.2018
(51) Int. Cl.: C07K 16/28, A61K 39/395, C07K 1/00, C07K 1/107

(54) **MONOMERIC HUMAN IgG1 Fc AND BISPECIFIC ANTIBODIES**
MONOMERE HUMANE IGG1-FC- UND BISPEZIFISCHE ANTIKÖRPER
ANTICORPS BISPÉCIFIQUES ET FC D'IGG1 HUMAINE MONOMÈRES

(30) Priority: 01.02.2017 US 201762453451 P; 26.07.2017 US 201762537415 P
(43) Date of publication of application: 11.12.2019
(73) Proprietor: Zhejiang Shimai Pharmaceutical Co., Ltd., Binjiang Hangzhou (CN)
(72) Inventor: CHEN, Weizao, Frederick Maryland 21704 (US); XIAO, Zuoxiang, Frederick Maryland 21704 (US); FU, Tao, Frederick Maryland 21704 (US)
(74) Representative: Grund, Martin
(86) International application number: PCT/US2018/016524
(87) International publication number: WO 2018/144784

(56) References cited:
- WO-A1-2011/063348
- WO-A1-2011/063348
- WO-A1-2013/138643
- WO-A1-2016/179707
- US-A1- 2006 074 225
- US-A1- 2015 050 278
- LU SHAN ET AL: "Generation and Characterization of an IgG4 Monomeric Fc Platform", PLOS ONE, vol. 11, no. 8, 1 August 2016 (2016-08-01), pages e0160345, XP055627509, DOI: 10.1371/journal.pone.0160345
- HONGYAN LIU ET AL: "Fc Engineering for Developing Therapeutic Bispecific Antibodies and Novel Scaffolds", FRONTIERS IN IMMUNOLOGY, vol. 8, 26 January 2017 (2017-01-26), XP055396345, DOI: 10.3389/fimmu.2017.00038
- SPIESS CHRISTOPH ET AL: "Alternative molecular formats and therapeutic applications for bispecific antibodies", MOLECULAR IMMUNOLOGY, PERGAMON, GB, vol. 67, no. 2, 27 January 2015 (2015-01-27), pages 95 - 106, XP029246892, ISSN: 0161-5890, DOI: 10.1016/J.MOLIMM.2015.01.003
- MA JIABING ET AL: "Bispecific Antibodies: From Research to Clinical Application.", FRONTIERS IN IMMUNOLOGY 2021, vol. 12, 2021, pages 626616, ISSN: 1664-3224
- CAO MINGYAN ET AL: "Characterization and Monitoring of a Novel Light-heavy-light Chain Mispair in a Therapeutic Bispecific Antibody", JOURNAL OF PHARMACEUTICAL SCIENCES, vol. 110, no. 8, August 2021 (2021-08-01), pages 2904 - 2915, ISSN: 0022-3549(print)
- SIMON KRAH ET AL: "Engineering IgG-Like Bispecific Antibodies-An Overview", ANTIBODIES, vol. 7, no. 3, 1 August 2018 (2018-08-01), CH, pages 28, XP055602846, ISSN: 2073-4468, DOI: 10.3390/antib7030028
- ULRICH BRINKMANN ET AL: "The making of bispecific antibodies", MABS, vol. 9, no. 2, 10 January 2017 (2017-01-10), US, pages 182 - 212, XP093126617, ISSN: 1942-0862, DOI: 10.1080/19420862.2016.1268307
- YING ET AL.: "Engineered Soluble Monomeric IgG1 CH 3 Domain generation, mechanisms of function, and implications for design of biological therapeutics", THE JOURNAL OF BIOLOGICAL CHEMISTRY, vol. 288, no. 35, 30 August 2013 (2013-08-30), pages 25154 - 25164, XP055118935

## Description

### BACKGROUND OF THE INVENTION

Antibodies play a central role in defense against invading non-self molecules. Antibodies' ability to interact with neonatal Fc-receptor (FcRn) through Fc (Fragment, Crystallizable) region in a pH-dependent manner confers them with extended serum half-life (Ghetie and Ward 2000). This unique feature of antibodies allows extending the half-life of therapeutic protein or peptide in the serum by engineering Fc-fusion molecules. Naturally occurring IgG antibodies and the engineered Fc-fusion molecules are bivalent and monospecific. This is due to the homodimeric nature of the antibody Fc. For certain therapeutic applications, it would be desirable to retain all the positive attributes conferred by the antibody or the Fc fragment of the antibody, meanwhile to achieve flexibility and specificity by engineering monomeric Fc (mFc) polypeptides.

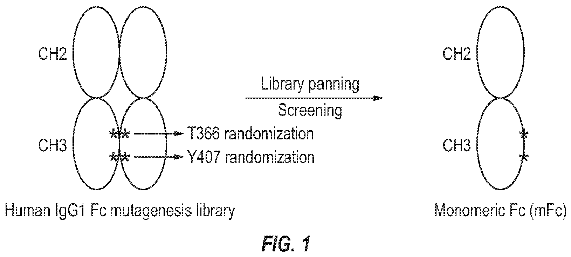

The most abundant immunoglobulin class in human serum is IgG. The IgG structure has four chains, two light and two heavy chains; each light chain has two domains and each heavy chain has four domains. The antigen binding site is located in the ab region (Fragment antigen binding) which contains a variable light (VL) and a variable heavy (VH) chain domain as well as constant light (CL) and constant heavy (CH1) chain domains. The Fc (Fragment, Crystallizable) region of the antibody contains CH2 and CH3 domain region of the heavy chain (FIG. 1). The IgG molecule can be considered as a heterotetramer having two heavy chains that are held together by disulfide bonds (-S-S-) at the hinge region and two light chains. The FcRn (neonatal Fc receptor) binding site of IgG is located in the Fc region of the antibody (Martin, West et al. 2001), and thus the extended serum half-life property of the antibody is retained in the Fc fragment. The Fc fragment alone can be thought of as a homodimer of heavy chains comprising CH2 and CH3 domains. A monovalent antibody with half the size of a full antibody includes only one light and one heavy chain with some mutations in the heavy chain Fc region to stabilize antibody in aqueous solution/serum. Monovalent IgGs with three or more mutations in the Fc region have been successfully developed for targeting cancer biomarkers (Merchant, Ma et al. 2013). WO 2013/138643 A1, US 2006/074225 and Shan et al. (PLOS One 2016, 11(8):e0160345) disclose Fc polypeptides with substitutions at positions including T366 and Y407. Monovalent IgG with only two mutations in the Fc region could lead to no or less immunogenicity in humans compared to Fc with three or more mutations.

With the recent advance of genetic and protein engineering technologies, bispecific antibodies (BsAb) such as BiTE, Xmab and CrossMab bispecific technology emerged to show promising applications (Nagorsen, Bargou et al. 2009) (Schaefer, Regula et al. 2011). Two BsAbs are approved for therapy and more than thirty are in clinical development. BiTE is a type of fusion proteins with two single-chain antibody variable fragments (scFvs) linked by a (G4S)3 polypeptide linker. In the absence of Fc, they cannot be purified with protein A and G, and have short in vivo half-lives, and therefore, continuous infusion is required in clinical use. Roche CrossMab contains Fc and therefore has much longer half-life in vivo than BiTE. CrossMab uses the knob-in-hole technology for Fc heterodimerization, but it could not yield 100% heterodimeric antibodies. Xmab bispecific technology uses a combination of CH1/CL interaction and electrostatic interaction at CH3 domains to form two antigen binding sites at the N terminals and C terminals of the two Fc polypeptides respectively. But an immune synapse similar to those formed in the course of natural cytotoxic T cell recognition is not easily formed due to the long distance of two binding site of Xmab at the N terminals and C terminals. There thus remains a pressing need to make a new type of antibodies such as engineered bispecific antibodies to improve the treatment of cancer or other diseases.

### SUMMARY OF THE INVENTION

The present invention provides monomeric Fc (mFc) polypeptides comprising a sequence selected from the group consisting of SEQ ID NOs: 9, 13, 15, and 17, which have a CH3 domain comprising one or two amino acid substitutions. The substitutions in the present invention significantly reduce the ability of the polypeptides to form homodimers. In one embodiment, the monomeric Fc polypeptides are non-natural occurring polypeptides. The present invention also provides various configuration of bispecific antibodies comprising an mFc polypeptide.

The mFc polypeptides comprise an IgG CH3 domain wherein said CH3 domain comprises amino acid substitutions at T366 and Y407. The T366 is substituted with L (Leucine). Y407 is substituted with H(Histidine), T(Threonine), A(Alanine), or N (Asparagine).

In another embodiment, the monomeric Fc polypeptides with the substituted CH3 forms have less immunogenicity or no immunogenicity than the ones with three or more amino acid substitutions in the CH3 domain. The monomeric Fc and its variants are selected from a group consisting of SEQ ID NO:9, SEQ ID NO:13, SEQ ID NO:15, and SEQ ID NO: 17.

The monomeric Fc (mFc) polypeptide is further linked to an antibody variable domain or is comprised within an antibody heavy chain. In certain embodiments, a monomeric antibody comprises the monomeric heavy chain and a light chain, essentially creating a half-antibody. In another embodiment, the monomeric heavy chain comprises one or more mutated cysteine residues to prevent disulfide bond formation. Particularly useful cysteine mutations are those in the hinge region of the heavy chain.

In one embodiment, the Fc polypeptides could have less immunogenicity compared to an Fc polypeptide comprising three or more substitutions.

Other aspects of the invention are non-nature occurring polynucleotide sequences encoding monomeric Fc polypeptides of the invention. In certain embodiments, an isolated DNA encodes a polynucleotide sequence comprising SEQ ID NO: 9, SEQ ID NO: 13, SEQ ID NO: 15, or SEQ ID NO: 17. In another embodiment, the invention provides an expression vector comprising such nucleic acids, and host cells comprising such expression vectors to express the polypeptides.

Embodiments of the invention further comprise methods of preparing a monomeric Fc polypeptide of the invention. In one embodiment, the methods comprise culturing host cells transiently or stably transfected with DNA encoding a monomeric Fc polypeptide under conditions wherein the monomeric Fc polypeptide is expressed, and then recovering the monomeric Fc polypeptide from the host cell culture.

The invention further comprises methods of making a bispecific antibody format with CH1/CL for heterodimerization and with mFc for extended half-life. The present invention provides a design of the bispecific antibody format comprising an mFc described above, wherein such bispecific antibody has an extended half-life with possibly less immunogenicity, and no interruption of the heterodimerization strength of CH1 and CL.

In one embodiment, provided herein is a bispecific monomeric Fc antibody (Bi-mFc) comprising: (a) a polypeptide chain having the formula V1-L1-V2-L2-V3 -L3-V4-L4-mFc, wherein mFc is an mFc polypeptide chain of the invention, wherein V1, V2, V3, and V4 are immunoglobulin variable regions that have the same or different amino acid sequences, wherein L1, L2, L3, and L4 are linkers, and wherein L4 can be present or absent; or (b) a polypeptide chain having the following formula: mFc-L4-Vl-L1-V2-L2-V3-L3-V4, wherein the mFc is a monomeric Fc polypeptide chain of the invention, wherein V1, V2, V3, and V4 are immunoglobulin variable regions that have the same or different amino acid sequences, wherein L1, L2, L3, and L4 are linkers, and wherein L4 can be present or absent; or (c) a polypeptide chain having the following formula: Vl-L1-V2-L2-mFc-L4 -V3-L3-V4, wherein mFc is a monomeric Fc polypeptide chain of the invention, wherein V1, V2, V3, and V4 are immunoglobulin variable regions that have the same or different amino acid sequences, wherein L1, L2, L3, and L4 are linkers, and wherein L2 can be present or absent.

The Bi-mFc comprises a monomer wherein the Bi-mFc mediates cytolysis of a target cell displaying a target cell protein by an immune effector cell, and does not mediate cytolysis of a cell not displaying the target cell protein by the immune effector cell. The Bi-mFc binds to a target cell and/or to an immune effector cell. In another embodiment, the mFc polypeptide chain of (a), (b) or (c) comprise one or more substitutions that inhibits FcyR binding, including but not limited to one or more of L234A, L235A, and any substitution at N297.

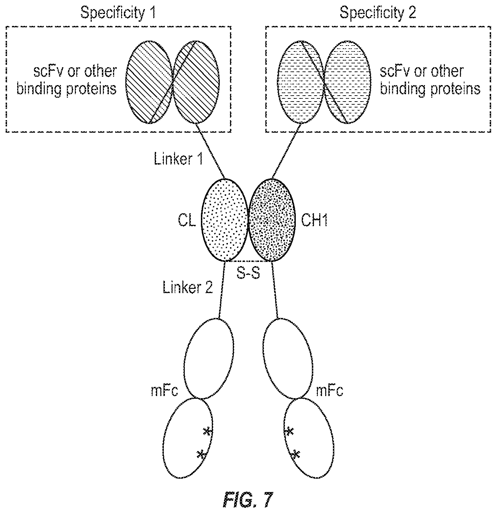

In one embodiment, the present invention provides a bispecific antibody comprising a monomeric Fc fusion protein comprising a CH3 domain with the two amino acid substitutions as described above. In one embodiment, the present invention provides a bispecific antibody comprising a monomeric Fc fusion protein comprising CH2 and CH3 domains wherein the CH3 domain consists of two amino acid substitutions as described above, wherein the two amino acid substitutions are at T366 and Y407. In yet another embodiment, the present invention comprises a bispecific antibody comprising two different chains wherein 1) one chain comprises an mFc with one or two amino acid substitutions on CH3 (described above) wherein its N-terminus is connected to CL by a linker, and the N-terminus of CL is connected to a single chain variable fragment (scFv) by another linker; 2) the other chain comprises the mFc of 1) wherein its N-terminus is connected to CH1 by a linker, and the N-terminus of CH1 is connected to the same or another scFv by another linker; and 3) the two different chains are connected by the heterodimerization of CL and CH1. In yet another embodiment, the present invention comprises a bispecific antibody similar to the above mentioned, wherein a scFv can also be linked to the C-terminus of mFc. In another further embodiment, the heterodimerization is enhanced by a disulfide bond or bonds (FIG. 7).

The present invention further provides a bispecific antibody format iBiBody, comprising a binding protein, a Fab and one or more monomeric Fc polypeptide, wherein the said binding protein is linked to an N terminal or C-terminal of said Fab and wherein the said one or more monomeric Fc polypeptides are linked to the other terminals of said Fab.

The present invention provides a design of the bispecific antibody format iBiBody comprising an mFc described above, wherein such bispecific antibody has an extended half-life with possibly less or no immunogenicity, and no interruption of the heterodimerization strength of VH-CH1 and VL-CL of a Fab. In certain embodiments, shortened hinge and mFc are used, which retains binding to the neonatal FcR ("good FcR") which mediates long half-life of antibodies while decreasing or eliminating binding to most other FcR ("bad FcR"). An iBiBody bispecific antibody comprising monomeric Fc has the advantages of longer half-life and high level of expression. It is easy to construct and easy to purify.

In one embodiment, the binding protein of iBiBody can be an antibody fragment (such as Fab, scFv, diabody, variable domain derived binders, nanobody). In one embodiment, the binding protein of iBiBody is an alternative scaffold derived protein binding domains (such as Fn3 variants, ankyrin repeat variants, centyrin variants, avimers, affibody) recognizing specific antigens. In one embodiment, the binding protein of iBiBody is a natural soluble ligand or receptor. In another embodiment, the binding protein of iBiBody is any protein that binds to another entity.

### BRIEF DESCRIPTION OF THE DRAWINGS

FIG. 1. Design of human IgG1 Fc mutagenesis library and selection of monomeric Fc (mFc).
FIG. 2. Identification of seven first-generation mFc variants with preserved binding to protein A and G.
FIG. 3. Expression and purification of the seven mFc variants.
FIG. 4. Oligomeric state of the first generation mFc variants in PBS (pH7.4).
FIG. 5. Identification of nine second-generation mFc variants by randomization of the M407 residue of mFc7.
FIG. 6. Oligomeric state of the second-generation mFc variants in PBS (pH7.4).
FIG. 7. A novel bispecific antibody format with CH1/CL for heterodimerization and mFc for extended half-life.
FIG. 8. Generation of a proof-of-concept bispecific antibody.
FIG. 9. Size-exclusion chromatography (SEC) of the purified bispecific antibody. SEC revealed that the KappaSelect-purified bispecific antibody contained dissociated scFv 1-CK-mFc7.x monomer, heterodimer and higher-order oligomers which could be well separated from each other by SEC leading to a relatively pure heterodimer of scFv 1-CK-mFc7.x/scFv 2-CH1-mFc7.x.
FIG. 10(a) - 10(c). Examples of bispecific antibody formats with only one polypeptide chain.
FIG. 11. Examples of "i-shaped" bispecific antibody format (iBiBody) with mFc.

### DETAILED DESCRIPTION OF THE INVENTION

### DEFINITION

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well-known and commonly used in the art. The methods and techniques of the present invention are generally performed according to conventional methods well known in the art and as described in various general and more specific references that are cited and discussed throughout the present specification unless otherwise indicated. See, e.g., Sambrook et al. Molecular Cloning: A Laboratory Manual, 2nd ed., Cold Spring Harbor Laboratory Press, Cold Spring Harbor, N.Y. (1989) and Ausubel et al, Current Protocols in Molecular Biology, Greene Publishing Associates (1992), and Harlow and Lane Antibodies: A Laboratory Manual Cold Spring Harbor Laboratory Press, Cold Spring Harbor, N.Y. (1990). Enzymatic reactions and purification techniques are performed according to manufacturer's specifications, as commonly accomplished in the art or as described herein. The terminology used in connection with, and the laboratory procedures and techniques of, analytical chemistry, synthetic organic chemistry, and medicinal and pharmaceutical chemistry described herein are those well-known and commonly used in the art. Standard techniques can be used for chemical syntheses, chemical analyses, pharmaceutical preparation, formulation, and delivery, and treatment of patients.

The following terms, unless otherwise indicated, shall be understood to have the following meanings: The term "isolated molecule" (where the molecule is, for example, a polypeptide, a polynucleotide, or an antibody) is a molecule that by virtue of its origin or source of derivation (1) is not associated with naturally associated components that accompany it in its native state, (2) is substantially free of other molecules from the same species (3) is expressed by a cell from a different species, or ( 4) does not occur in nature. Thus, a molecule that is chemically synthesized, or expressed in a cellular system different from the cell from which it naturally originates, will be "isolated" from its naturally associated components. A molecule also may be rendered substantially free of naturally associated components by isolation, using purification techniques well known in the art. Molecule purity or homogeneity may be assayed by some means well known in the art. For example, the purity of a polypeptide sample may be assayed using polyacrylamide gel electrophoresis and staining of the gel to visualize the polypeptide using techniques well known in the art. For certain purposes, higher resolution may be provided by using HPLC or other means well known in the art for purification.

Polynucleotide or nucleic acid and polypeptide sequences are indicated using standard one- or three-letter abbreviations. Unless otherwise indicated, polypeptide sequences have their amino termini at the left and their carboxyl termini at the right, and single-stranded nucleic acid sequences, and the top strand of double-stranded nucleic acid sequences have their 5' termini at the left and their 3' termini at the right. A particular polypeptide or polynucleotide sequence also can be described by explaining how it differs from a reference sequence.

The terms "peptide" "polypeptide" and "protein" each refers to a molecule comprising two or more amino acid residues joined to each other by peptide bonds. These terms encompass, e.g., native and artificial proteins, protein fragments and polypeptide analogs (such as mutations, variants, and fusion proteins) of a protein sequence as well as post-translationally, or otherwise covalently or non-covalently, modified proteins. A peptide, polypeptide, or protein may be monomeric or polymeric.

The term "polypeptide fragment" as used herein refers to a polypeptide that has an amino-terminal and/or carboxyl-terminal deletion as compared to a corresponding full-length protein. Fragments can be, for example, at least 5, 6, 7, 8, 9, 10, 11, 12, 13, 14, 15, 20, 50, 70, 80, 90,100,150, 200, 250, 300, 350, or 400 amino acids in length. Fragments can also be, for example, at most 1,000, 750, 500, 250, 200, 175,150,125, 100, 90, 80, 70, 60, 50, 40, 30, 20, 15, 14, 13, 12, 11, or 10 amino acids in length. A fragment can further comprise, at either or both of its ends, one or more additional amino acids, for example, a sequence of amino acids from a different naturally-occurring protein or an artificial amino acid sequence.

Polypeptides include polypeptides that have been modified in any way and for any reason, for example, to: (1) reduce susceptibility to proteolysis, (2) reduce susceptibility to oxidation, (3) alter binding affinity for forming protein complexes, (4) alter binding affinities, and ( 4) confer or modify other physicochemical or functional properties. Analogs include mutations of a polypeptide. For example, single or multiple amino acid substitutions (e.g., conservative amino acid substitutions) may be made in the naturally occurring sequence (e.g., in the portion of the polypeptide outside the domain(s) forming intermolecular contacts). A "conservative amino acid substitution" is one that does not substantially change the structural characteristics of the parent sequence (e.g., a replacement amino acid should not tend to break a helix that occurs in the parent sequence, or disrupt other types of secondary structure that characterize the parent sequence or are necessary for its functionality).

Examples of art-recognized polypeptide secondary and tertiary structures are described in Proteins, Structures and Molecular Principles (Creighton, Ed., W. H. Freeman and Company, New York (1984)); Introduction to Protein Structure (C. BrandenandJ. Tooze, eds., Garland Publishing, New York, N.Y. (1991)); and Thornton et al. Nature 354:105 (1991).

A "variant" of a polypeptide comprises an amino acid sequence wherein one or more amino acid residues are inserted into, deleted from or substituted into the amino acid sequence relative to another polypeptide sequence. Variants of the invention include those comprising a variant CH2 or CH3 domain. In certain embodiments, a variant comprises one or more mutations that when present in an Fc molecule increase affinity for the polypeptide to one or more FcRns.

Such variants demonstrate enhanced antibody-dependent cell-mediated cytotoxicity. Examples of variants providing such are described in U.S. Pat. No. 7,317,091.

Other variants include those that decrease the ability of CH3-domain containing polypeptides to homodimerize. Examples of such Fc variants are described in US 5,731,168 and 7,183,076, 9,493,578 and 9,200,060.

A "derivative" of a polypeptide is a polypeptide (e.g., an antibody) that has been chemically modified, e.g., via conjugation to another chemical moiety such as, for example, polyethylene glycol, a cytotoxic agent, albumin (e.g., human serum albumin), phosphorylation, and glycosylation. Unless otherwise indicated, the term "antibody" includes, in addition to antibodies comprising two full-length heavy chains and two full-length light chains, derivatives, variants, fragments, and mutations thereof, examples of which are described herein.

The term "antibody" as meant herein, is a protein or polypeptide containing at least one VH or VL region, in many cases a heavy and a light chain variable region. Thus, the term "antibody" encompasses molecules having a variety of formats, including single chain Fv antibodies (scFv, which contain VH and VL regions joined by a linker), Fab, F(ab)2 ', Fab', scFv:Fc antibodies (as described in Carayannopoulos and Capra, Ch. 9 in FUNDAMENTAL IMMUNOLOGY, 3rd ed., Paul, ed., Raven Press, New York, 1993, pp. 284-286), or full-length antibodies containing two full length heavy and two full-length light chains, such as naturally-occurring IgG antibodies found in mammals. The Fc domain can be monomeric or dimeric. An antibody can be "monomeric," i.e., comprising a single polypeptide chain. An antibody can comprise multiple polypeptide chains ("multimeric") or can comprise two ("dimeric"), three ("trimeric"), or four ("tetrameric") polypeptide chains. An antibody can be chimeric. The light chain and heavy chain of the natural antibody have CL and CH1 domain respectively, which forms specific CH1/CL interaction.

The term "bispecific antibody" is an antibody binding to two different antigens.

The term "bispecific monomeric Fc antibody" (Bi-mFc) as meant herein, comprises a first polypeptide chain with an mFc and, optionally, a second polypeptide chain. In many embodiments, a Bi-mFc comprises both a first and a second polypeptide chain. In some embodiments, a Bi-mFc is a monomer comprising only the first polypeptide chain. The first polypeptide chain comprises two VH regions and two VL regions separated by linkers and an Fc polypeptide chain. The Fc polypeptide chain can be N-terminal or C-terminal relative to the four immunoglobulin variable regions, and it can be joined to the variable regions via a linker. This linker can be present or absent. The second polypeptide chain, if present, comprises an mFc polypeptide chain. Thus, a Bi-mFc can be a monomer or a heterodimer.

The term "iBiBody" is a bispecific antibody comprising a binding protein, a Fab and one or more monomeric Fc polypeptide, wherein the said binding protein is linked to an N terminal or C-terminal of said Fab and wherein the said one or more monomeric Fc polypeptides are linked to the other terminals of said Fab. The said binding protein and Fab bind to two different antigens.

The term "hydrophobic residue" or "hydrophobic amino acid" as meant herein includes amino acids that have hydrophobic side chains including but not limited to glycine (G), alanine (A), valine (V), leucine (L), isoleucine (I), proline (P), phenylalanine (F), methionine (M), and tryptophan (W).

The term "human antibody" includes all antibodies that have one or more variable and constant regions derived from human immunoglobulin sequences. In one embodiment, all of the variable and constant domains are derived from human immunoglobulin sequences (a fully human antibody).

These antibodies may be prepared in a variety of ways, including through the immunization with an antigen of interest of a mouse that is genetically modified to express antibodies derived from human heavy and/or light chain-encoding genes. In certain embodiments, the heavy chain of a human antibody is altered in the CH3 domain to reduce the ability of the heavy chain to dimerize.

A humanized antibody has a sequence that differs from the sequence of an antibody derived from a non-human species by one or more amino acid substitutions, deletions, and/or additions, such that the humanized antibody is less likely to induce an immune response, and/or induces a less severe immune response, as compared to the non-human species antibody, when it is administered to a human subject.

In one embodiment, certain amino acids in the framework and constant domains of the heavy and/or light chains of the non-human species antibody are mutated to produce the humanized antibody. In another embodiment, the constant domain(s) from a human antibody are fused to the variable domain(s) of a non-human species. Examples of how to make humanized antibodies may be found in U.S. Pat. Nos. 6,054, 297, 5,886,152 and 5,877,293.

The term "chimeric antibody" refers to an antibody that contains one or more regions from one antibody and one or more regions from one or more other antibodies. In one example of a chimeric antibody, a portion of the heavy and/or light chain is identical with, homologous to, or derived from an antibody from a particular species or belonging to a particular antibody class or subclass, while the remainder of the chain(s) is/are identical with, homologous to, or derived from an antibody (-ies) from another species or belonging to another antibody class or subclass. Also included are fragments of such antibodies that exhibit the desired biological activity.

Fragments or analogs of antibodies can be readily prepared by those of ordinary skill in the art following the teachings of this specification and using techniques well known in the art. Preferred amino- and carboxyl-termini of fragments or analogs occur near boundaries of functional domains. Structural and functional domains can be identified by comparison of the nucleotide and/or amino acid sequence data to public or proprietary sequence databases. Computerized comparison methods can be used to identify sequence motifs or predicted protein conformation domains that occur in other proteins of known structure and/or function. Methods to identify protein sequences that fold into a known three-dimensional structure are known. See, e.g., Bowie et al.1991, Science 253:164.

A "CDR grafted antibody" is an antibody comprising one or more CDRs derived from an antibody of a particular species or isotype and the framework of another antibody of the same or different species or isotype.

The "percent identity" of two polynucleotide or two polypeptide sequences is determined by comparing the sequences using the GAP computer program (a part of the GCG Wisconsin Package, version 10.3 (Accelrys, San Diego, Calif)) using its default parameters.

The terms "polynucleotide," "oligonucleotide" and "nucleic acid" are used interchangeably throughout and include DNA molecules (e.g., cDNA or genomic DNA), RNA molecules (e.g., mRNA), analogs of the DNA or RNA generated using nucleotide analogs (e.g., peptide nucleic acids and non-naturally occurring nucleotide analogs), and hybrids thereof. The nucleic acid molecule can be single-stranded or double-stranded. In one embodiment, the nucleic acid molecules of the invention comprise a contiguous open reading frame encoding an antibody or an Fc-fusion, and a derivative, mutation, or variant thereof.

Two single-stranded polynucleotides are "the complement" of each other if their sequences can be aligned in an anti-parallel orientation such that every nucleotide in one polynucleotide is opposite its complementary nucleotide in the other polynucleotide, without the introduction of gaps, and without unpaired nucleotides at the 5' or the 3' end of either sequence. A polynucleotide is "complementary" to another polynucleotide if the two polynucleotides can hybridize to one another under moderately stringent conditions.

Thus, a polynucleotide can be complementary to another polynucleotide without being its complement.

A "vector" is a nucleic acid that can be used to introduce another nucleic acid linked to it into a cell. One type of vector is a "plasmid," which refers to a linear or circular double-stranded DNA molecule into which additional nucleic acid segments can be ligated. Another type of vector is a viral vector (e.g., replication defective retroviruses, adenoviruses and adeno-associated viruses), wherein additional DNA segments can be introduced into the viral genome. Certain vectors are capable of autonomous replication in a host cell into which they are introduced (e.g., bacterial vectors comprising a bacterial origin of replication and episomal mammalian vectors). Other vectors (e.g., non-episomal mammalian vectors) are integrated into the genome of a host cell upon introduction into the host cell and thereby are replicated with the host genome. An "expression vector" is a type of vector that can direct the expression of a chosen polynucleotide.

A nucleotide sequence is "operably linked" to a regulatory sequence if the regulatory sequence affects the expression (e.g., the level, timing, or location of expression) of the nucleotide sequence. A "regulatory sequence" is a nucleic acid that affects the expression (e.g., the level, timing, or location of expression) of a nucleic acid to which it is operably linked. The regulatory sequence can, for example, exert its effects directly on the regulated nucleic acid, or through the action of one or more other molecules (e.g., polypeptides that bind to the regulatory sequence and/or the nucleic acid). Examples of regulatory sequences include promoters, enhancers and other expression control elements (e.g., polyadenylation signals). Further examples of regulatory sequences are described in, for example, Goeddel, 1990, Gene Expression Technology: Methods in Enzymology 185, Academic Press, San Diego, Calif and Baron et al., 1995, Nucleic Acids Res. 23:3605-06.

A "host cell" is a cell that can be used to express a nucleic acid, e.g., a nucleic acid of the invention. A host cell can be a prokaryote, for example, *E. coli,* or it can be a eukaryote, for example, a single-celled eukaryote (e.g., a yeast or other fungus), a plant cell (e.g., a tobacco or tomato plant cell), an animal cell (e.g., a human cell, a monkey cell, a hamster cell, a rat cell, a mouse cell, or an insect cell) or a hybridoma. Exemplary host cells include Chinese hamster ovary (CHO) cell lines or their derivatives including CHO strain DXB-11, which is deficient in DHFR (see Urlaub et al, 1980, Proc. Natl. Acad. Sci. USA 77:4216-20), CHO cell lines which grow in serum-free media (see Rasmussen et al, 1998, Cytotechnology 28:31), CS-9 cells, a derivative of DXB-11 CHO cells, and AM-1/D cells (described in U.S. Pat. No. 6,210,924). Other CHO cells lines include CHO-Kl (ATCC# CCL-61), EM9 (ATCC# CRL-1861), and UV20 (ATCC# CRL-1862). Examples of other host cells include COS-7 line of monkey kidney cells (ATCC CRL 1651) (see Gluzman et al, 1981, Cell23:175), L cells, C127 cells, 3T3 cells (ATCC CCL 163), HeLa cells, BHK (ATCC CRL 10) cell lines, the CVl/EBNA cell line derived from the African green monkey kidney cell line CVl (ATCC CCL 70) (see McMahanetal., 1991,EMBOJ. 10:2821), human embryonic kidney cells such as 293, 293 EBNA or MSR 293, human epidermal A431 cells, human Colo205 cells, other transformed primate cell lines, normal diploid cells, cell strains derived from in vitro culture of primary tissue, primary explants, HL-60, U937, HaK or Jurkat cells. Typically, a host cell is a cultured cell that can be transformed or transfected with a polypeptide-encoding nucleic acid, which can then be expressed in the host cell.

The phrase "recombinant host cell" can be used to denote a host cell that has been transformed or transfected with a nucleic acid to be expressed. A host cell also can be a cell that comprises the nucleic acid but does not express it at a desired level unless a regulatory sequence is introduced into the host cell such that it becomes operably linked with the nucleic acid. It is understood that the term host cell refers not only to the particular subject cell but to the progeny or potential progeny of such a cell. Because certain modifications may occur in succeeding generations due to, e.g., mutation or environmental influence, such progeny may not, in fact, be identical to the parent cell, but are still included within the scope of the term as used herein.

"Binding protein" includes natural protein binding domains, antibody fragments (such as Fab, scFv, diabody, variable domain derived binders, nanobody), alternative scaffold derived protein binding domains (such as Fn3 variants, ankyrin repeat variants, centyrin variants, avimers, affibody) or any protein recognizing specific antigens. A binding protein can be linked to an mFc polypeptide to form an mFc fusion protein.

Antigen-binding fragment (Fab) is an antigen-binding portion of an antibody comprising a VH, a VL, a CH1 and a CL domain. In preferred embodiments, VH is linked to CH1 and VL is linked to CL domain. In other embodiments, VH is linked to CL and VL is linked to CH1.

### Pharmaceutical Compositions

The polypeptides of the invention are particularly useful for formulation into pharmaceutical compositions. Such compositions comprise one or more additional components such as a physiologically acceptable carrier, excipient or diluent. Optionally, the composition additionally comprises one or more physiologically active agents, for example, as described below. In various particular embodiments, the composition comprises one, two, three, four, five, or six physiologically active agents in addition to one or more monomeric antibody and/or Fc-fusion protein of the present invention.

In one embodiment, the pharmaceutical composition comprises a monomeric antibody and/or Fc-fusion protein of the invention together with one or more substances selected from the group consisting of a buffer, an antioxidant such as ascorbic acid, a low molecular weight polypeptide (such as those having fewer than 10 amino acids), a protein, an amino acid, a carbohydrate such as glucose, sucrose or dextrins, a chelating agent such as EDTA, glutathione, a stabilizer, and an excipient. Neutral buffered saline or saline mixed with conspecific serum albumin are examples of appropriate diluents. In accordance with appropriate industry standards, preservatives such as benzyl alcohol may also be added. The composition may be formulated as a lyophilizate using appropriate excipient solutions (e.g., sucrose) as diluents.

Suitable components are nontoxic to recipients at the dosages and concentrations employed. Further examples of components that may be employed in pharmaceutical formulations are presented in Remington's Pharmaceutical Sciences, 16th Ed. (1980) and 20th Ed. (2000), Mack Publishing Company, Easton, Pa.

Kits for use by medical practitioners are provided including one or more monomeric antibody and/or Fc-fusion proteins of the invention and a label or other instructions for use in treating any of the conditions discussed herein. In one embodiment, the kit includes a sterile preparation of one or more monomeric antibody and/or Fc-fusion protein, which may be in the form of a composition as disclosed above, and may be in one or more vials.

Dosages and the frequency of administration may vary according to such factors as the route of administration, the particular monomeric antibody and/or Fc-fusion protein employed, the nature and severity of the disease to be treated, whether the condition is acute or chronic, and the size and general condition of the subject. Appropriate dosages can be determined by procedures known in the pertinent art, e.g., in clinical trials that may involve dose escalation studies.

A monomeric antibody and/or Fc-fusion protein of the invention may be administered, for example, once or more than once, e.g., at regular intervals over a period of time. In particular embodiments, a monomeric antibody and/or Fc fusion protein is administered over a period of at least once a month or more, e.g., for one, two, or three months or even indefinitely. For treating chronic conditions, long-term treatment is generally most effective. However, for treating acute conditions, administration for shorter periods, e.g., from one to six weeks, may be sufficient. In general, the monomeric antibody and/or Fc-fusion protein is administered until the patient manifests a medically relevant degree of improvement over baseline for the chosen indicator or indicators.

As is understood in the pertinent field, pharmaceutical compositions comprising the monomeric antibody and/or Fc-fusion protein of the invention are administered to a subject in a manner appropriate to the indication. Pharmaceutical compositions may be administered by any suitable technique, including but not limited to parenterally, topically, or by inhalation. If injected, the pharmaceutical composition can be administered, for example, via intra-articular, intravenous, intramuscular, intralesional, intraperitoneal or subcutaneous routes, by bolus injection, or continuous infusion.

Localized administration, e.g., at a site of disease or injury is contemplated, as are transdermal delivery and sustained release from implants. Delivery by inhalation includes, for example, nasal or oral inhalation, use of a nebulizer, inhalation of the monomeric antibody and/or Fc-fusion protein in aerosol form, and the like.

The wild-type Fc is homodimeric in nature and this feature is driven by the strong, high-affinity interaction that exists between the two CH3 domains. Described herein are monomeric Fc molecules and methods of making and using such molecules. Although the term "Fc" is typically thought of as a homodimer of polypeptides, the term as used herein, due to the unique properties of the polypeptides of the invention, will also include monomeric polypeptides which comprises a sequence of amino acids corresponding to the Fc portion of the heavy chain, i.e., containing a CH2 and CH3 domain (FIG. 1; e.g. SEQ ID NO: 1).

The term "monomeric Fc polypeptide" or "monomeric Fc" (mFc) as meant herein, is a monomeric polypeptide containing a CH2 and CH3 domain. The said CH2 domain and CH3 domain can be variants of wild-type Fc CH2 or CH3 domain.

The term "monomeric Fc fusion protein" as meant herein, is a monomeric fusion protein containing an mFc and another domain connected with the mFc.

The methods described herein demonstrate that by substituting residues at the CH3 domain interface it is possible to completely disrupt CH3/CH3 association yet maintain the stability of the molecule, thus achieving a monomeric Fc.

The monomeric nature of the altered Fc can be assessed by, e.g., Size Exclusion Chromatography (SEC) and Analytical Ultra Centrifugation (AUC). The substitutions accomplish two things--one is to hinder the homodimer formation of the CH3 domain, and the other is to stabilize the monomeric form of Fc.

Methodology for identifying amino acids making up a CH3/CH3 interface is disclosed in WO2009089004. A total of 48 antibody crystal structures which had co-ordinates corresponding to the Fc region were identified from the Protein Data Bank (PDB) (Bernstein, Koetzle, et al. 1977) using a structure based search algorithm (Ye and Godzik 2004).

The atomic coordinates of Fc were extracted from the crystal structure of a human IgG1 antibody (Protein Data Bank entry 1HZH). All hydrophobic residues at the Fc interface were represented by using the PyMOL molecular graphics system (version 1.5.0.4; Schrödinger, LLC).

According to the contact based method, interface residues are defined as residues whose side chain heavy atoms are positioned closer than a specified limit from the heavy atoms of any residues in the second chain. Though 4.5 A distance limit is preferred, one could also use longer distance limit (for example, 5.5 A) in order to identify the interface residues (Bahar and Jemigan 1997).

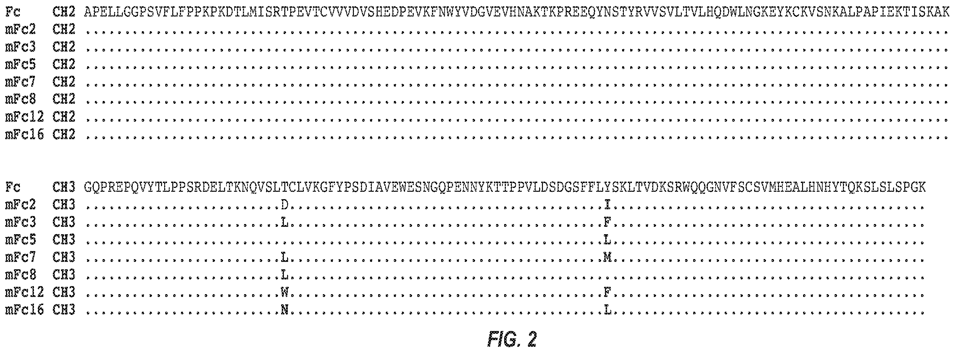

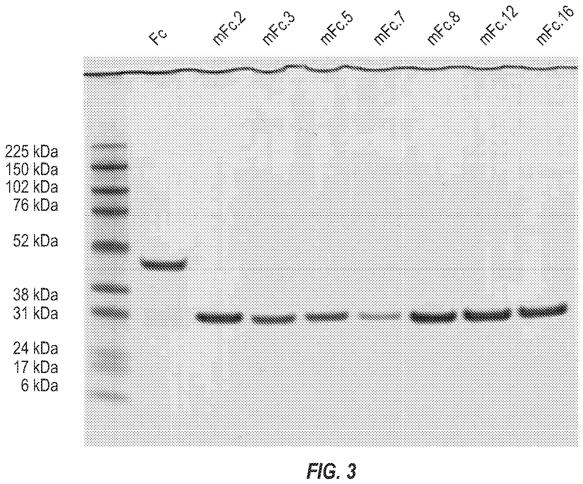

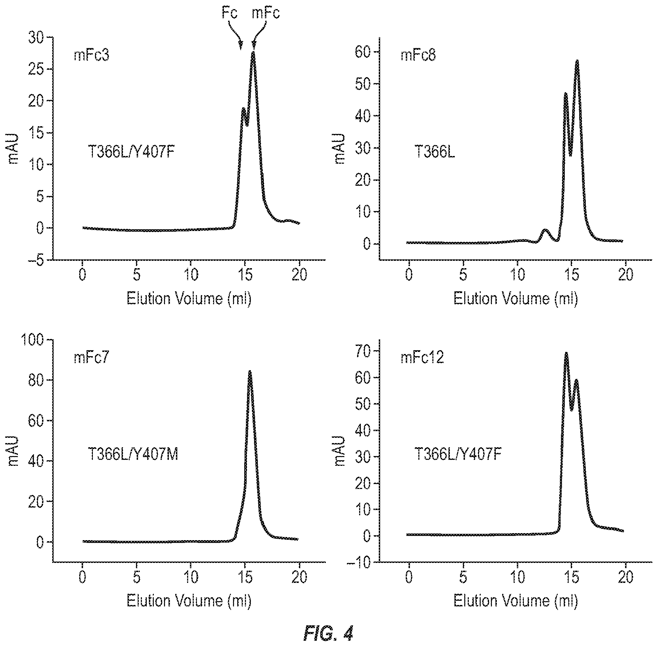

Various substitutions or mutations to the Fc portion of an antibody are described herein. Such variations are designated by the amino acid at that position in the wild-type antibody heavy chain based on the EU numbering scheme of Kabat followed by the amino acid substituted into that position. For example, when the tyrosine at EU position 407 is substituted with methionine, it is designated "Y407M." By "Fc," it is meant a wild-type sequence of amino acids that occurs naturally within a species of animals, e.g., humans. Wild-type sequence may vary slightly between individuals within a population, e.g., different alleles for the various immunoglobulin chains are known in the art. FIG. 2 lists seven mFc variants with one or two amino acid substitutions identified based on protein-G ELISA screening, using soluble expression-based monoclonal ELISA (semELISA). A hydrophobic residue substitution at position 407 of CH3 is preferred. In contrast, there is no preferential use of types of amino acid residues in position 366. Non-reducing SDS-PAGE and size-exclusion chromatography revealed that these mFc variants have a suboptimal oligomeric state in PBS (pH7.4) (FIG. 3 and 4).

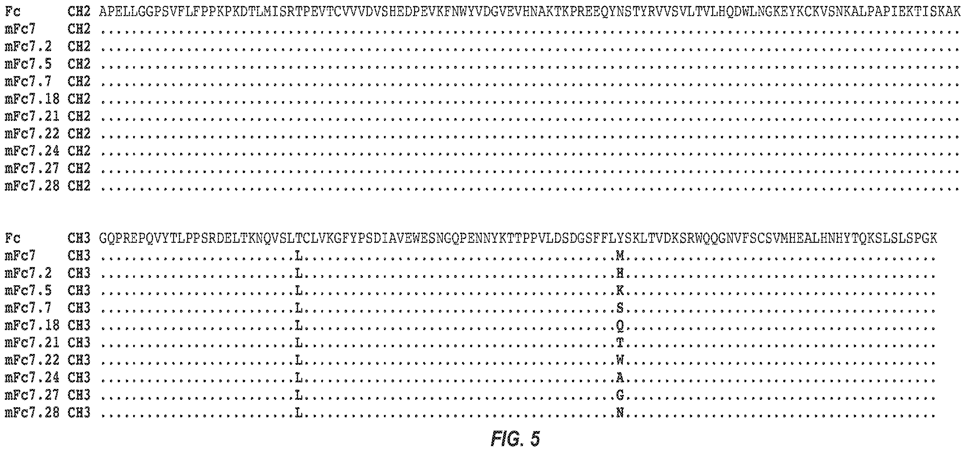

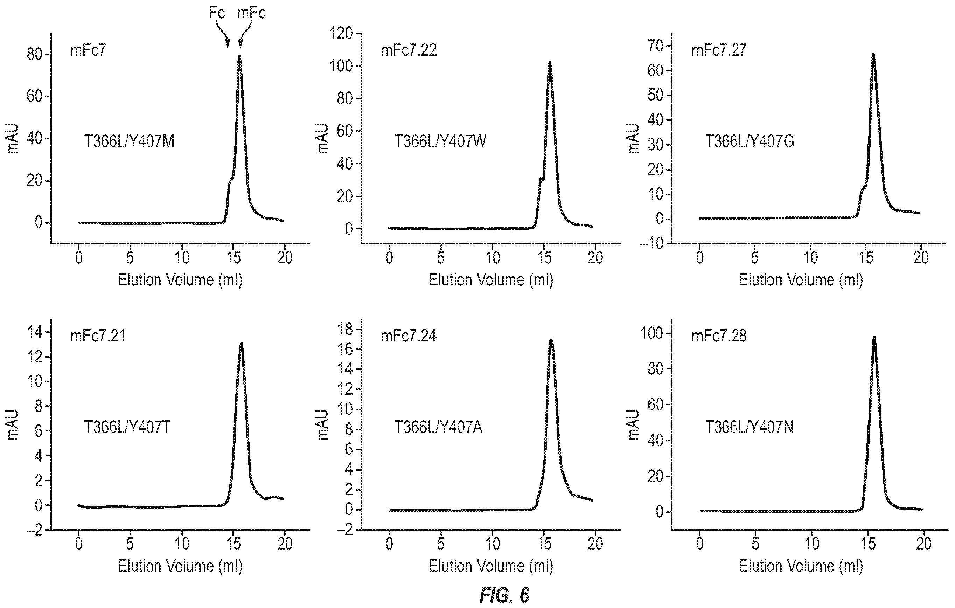

FIG. 5 lists nine second-generation mFc variants identified based on same protein-G ELISA screening by randomization of the Y407M residue of first-generation variant mFc7 (FIG. 5). The amino acid residue in position 366 is fixed as leucine. Size-exclusion chromatography revealed that more than 80% of the purified 9 second-generation mFc7.2, mFc7.5, mFc7.7, mFc7.18, mFc7.21, mFc7.24, mFc7.27 and mFc7.28 proteins in PBS (pH7.4) eluted as a monomer (some examples are in FIG. 6). There is no preferential use of types of amino acid residues in position 407 of the second-generation mFc variants.

In order to maintain the affinity to protein G and stability of the polypeptide in monomeric form, residue T366 can be replaced with leucine, while one of the large hydrophobic residues Y407 that make up the CH3/CH3 interface can be replaced with an amino acid choosing from histidine, alanine, or asparagine.

An antibody's ability to interact with neonatal Fc receptor (FcRn) in a pH-dependent manner confers it with extended serum half-life. In one embodiment, monomeric Fc molecules of the present invention retain the ability to bind FcRn similarly if not superiorly to wild-type Fc polypeptides.

For purposes of comparison of the characteristics of the CH3-containing molecules of the present invention to those of wild-type human CH3-containing molecules, the sequence of wild-type IgG1 CH3 domain is set forth in FIG. 2 SEQ ID NO: 1.

It is contemplated that the creation of monomeric Fc-containing molecules is not limited to those based on IgG1 Fc but are also applicable to the Fc region of IgG2, IgG3, IgG4 and other immunoglobulin subclasses including IgA, IgE, IgD, and IgM. The CH3 domain interface residues are highly conserved among IgGs. Therefore, the mutations that lead to monomerization in human IgG1 Fc can be extended to other human IgG subclasses as well as to species other than human.

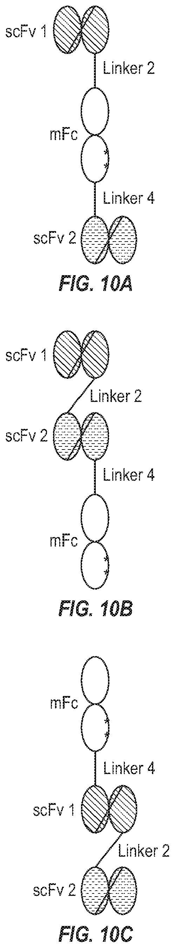

Virtually any molecule that contains an Fc domain may comprise a monomeric Fc domain of the present invention. Examples of such molecules are shown in FIG. 7 and 10. As seen in FIG. 7 and 10, various scFv peptides and binding proteins may be fused or linked to the N-terminus or C-terminus of the Fc.

In certain embodiments, the mFc polypeptide is linked to a Fab to create a half-antibody. Such half-antibody can be created by expressing a heavy chain comprising a monomeric Fc and a light chain recombinantly in a cell, e.g., CHO cell. The heavy chain may contain one or more further mutations. In certain embodiments, the heavy chain further comprises mutations of one or more cysteine residues in the hinge region (Allen et al., Biochemistry. 2009 May 5; 48 (17):3755-66).

The Fc polypeptides of the present invention demonstrate reduced homodimerization as compared to wild-type Fc molecules. Thus, embodiments of the invention include compositions comprising an antibody or Fc-fusion molecule wherein the amount of Fc-Fc homodimerization exhibited by said antibody or Fc-fusion molecule is less than 60%, less than 20%, less than 15%, less than 14%, less than 13%, less than 12%, less than 11%, less than 10%, less than 9%, less than 8%, less than 7%, less than 6%, less than 5%, less than 4%, less than 3%, less than 2%, or less than 1%. Dimerization may be measured by a number of techniques known in the art. Preferred methods of measuring dimerization include Size Exclusion Chromatography (SEC), Analytical Ultra Centrifugation (AUC), Dynamic Light Scattering (DLS), and Native PAGE.

The monomer Fc molecules described herein are useful for extending the half-life of therapeutic proteins or domains. Diseases that may be treated with an Fc monomer therapeutic may include inflammation, cancer, metabolic disorders, and others. Potential fusion targets include natural protein binding, antibody fragments (such as Fab, scFv, diabody, variable domain derived binders), and alternative scaffold derived protein binding domains (such as Fn3 variants, ankyrin repeat variants, centyrin variants, avimers, affibody and knottin) and peptides recognizing specific antigens. Fc monomer fusion proteins have the advantage of small in size, therefore potentially better ability to penetrate tissues. Fc monomer fusion proteins can be especially useful when monovalency of target binding is preferred. Such monovalency is often preferred when targeting cell-surface molecules that are susceptible to dimerization when targeted using multivalent antibodies.

Embodiments of the invention further include methods of preparing a bispecific antibody format with CH1/CL for heterodimerization and mFc for extended half-life. Besides retargeting effector cells to the site of cancer, new applications were established for bispecific antibodies. Bispecific antibodies that can simultaneously bind to cell surface antigens and payloads are an ideal delivery system for therapeutic and diagnostic use. Bispecific antibodies that can inhibit two correlated signaling molecules at the same time can be developed to overcome inherent or acquired resistance and to be more efficient angiogenesis inhibitors. Bispecific antibodies can also be used to treat hemophilia A by mimicking the function of factor VIII. Bispecific antibodies also have broad application prospects in bone disorders and infections and diseases of the central nervous system.

In another embodiment of heterodimerized bispecific antibodies, one of the monomeric Fc fusion proteins comprises a single-chain variable fragment (scFv), an mFc domain and a CL domain. In certain embodiments, the CL and scFv are connected by linker 1 while the mFc and CL are connected by linker 2. In certain embodiments, the linker 2 may have one or more cysteine residues to form disulfide bonds. The other monomeric Fc fusion protein comprises the same or another scFv, an mFc domain and a CH1 domain. In certain embodiments, the CH1 and scFv are connected by linker 1 while the mFc and CH1 are connected by linker 2. In certain embodiments, the linker 2 may have one or more cysteine residues to form disulfide bonds (FIG 7).

A bispecific antibody can also be a bispecific monomeric Fc antibody (Bi-mFc). Bi-mFc comprising a CH3 domain with one or two amino acid substitutions can bind monovalently to two different antigens. In addition, it can bind to the neonatal Fc receptor (FcRn) at slightly acidic pH (about pH5.5-6.0) via its Fc region. This interaction with FcRn can lengthen the half-life of a molecule in vivo. The first polypeptide chain (which, in some cases, is the only polypeptide chain) comprises an mFc domain and two VH regions plus two VL regions separated by linkers. The mFc domain can be N-terminal, C-terminal or in the middle relative to the four immunoglobulin variable regions, and it can be joined to the variable regions via a linker. The second polypeptide chain, when present, comprises an mFc polypeptide chain. A Bi-mFc can bind to an immune effector cell and a target cell and/or can mediate cytolysis of a target cell by an immune effector cell. The general structure of a Bi-mFc with only one polypeptide is diagrammed in FIG. 10, which shows an embodiment where the mFc domain is in the middle (a), and an embodiment where the mFc domain is at the C terminal (b) and an embodiment where the mFc domain is at the N-terminal (c). In one embodiment, a bispecific antibody comprises a scFv connecting with N-terminal of mFc via a linker and C-terminus of mFc is connected with the same or another scFv via a linker. In yet another embodiment, a bispecific antibody comprises an mFc wherein its N-terminus is linked to a scFv2 by a linker and then the same or another scFv is connected to the first scFv via another linker. In yet another embodiment, a bispecific antibody comprises an mFc wherein its C-terminus is connected to a scFv by a linker and the scFv is connected to the same or another scFv by another linker (FIG. 10).

More particular embodiments specify the order of immunoglobulin variable regions and the length of the linkers and specify which immunoglobulin variable regions can associate to form a binding site for an effector cell protein or a target cell protein. Generally, the antigen-binding portion of an antibody includes both a VH and a VL region, referred to herein as a "VH/VL pair," although in some cases a VH or a VL region can bind to an antigen without a partner. See, e.g., US Application Publication 2003/0114659. "VH/VL pair" can be connected by a linker to form a single chain variable domain (scFv).

In one group of embodiments, the four variable regions can be arranged in the following order: VH1-linkerl-VL1-linker2-VH2-linker3-VL2, where VH1/VL1 is an antigen-binding pair and VH2/VL2 is another antigen-binding pair. In this group of embodiments, linker 1 and linker3 can be at least 15 amino acids long, and linker2 can be less than 12 amino acids long. In some embodiments, the VH1/VL1 pair can bind to a target cell protein, and the VH2/VL2 pair can bind to an effector cell protein. In other embodiments, the VH1/VL1 pair can bind to an effector cell protein, and the VH2/VL2 pair can bind to a target cell protein.

In another group of embodiments, the four variable regions can be arranged in the following order: VL1-linkerl-VH1-linker2-VL2-linker3-VH2, where VH1VL1 is an antigen-binding pair and VH2VL2 is an antigen-binding pair. In these embodiments, linker2 can be less than 12 amino acids long, and linker1 and linker3 can be at least 15 amino acids long. In some embodiments, the VH1/VL1 pair can bind to a target cell protein, and the VH2/VL2 pair can bind to an effector cell protein. In other embodiments, the VH1/VL1 pair can bind to an effector cell protein, and the VH2/VL2 pair can bind to a target cell protein.

In another group of embodiments, the four variable regions can be arranged in the following order: VH1-linkerl-VL1-linker2-VL2-linker3-VH2, where VH1/VL1 is an antigen-binding pair and VH2/VL2 is an antigen-binding pair. In these embodiments, linker2 can be less than 12 amino acids long, and linker1 and linker3 can be at least 15 amino acids long. In some embodiments, the VH1/VL1 pair can bind to a target cell protein, and the VH2/VL2 pair can bind to an effector cell protein. In other embodiments, the VH1/VL1 pair can bind to an effector cell protein, and the VH2/VL2 pair can bind to a target cell protein.

In a further group of embodiments, the four variable regions can be arranged in the following order: VL1-linkerl-VH1-linker2-VH2-linker3-VL2, where VH1/VL1 is an antigen-binding pair and VH2/VL2 is an antigen-binding pair. In these embodiments, linker2 can be less than 12 amino acids long, and linker1 and linker3 can be at least 15 amino acids long. In some embodiments, the VH1/VL1 pair can bind to a target cell protein, and the VH2/VL2 pair can bind to an effector cell protein. In other embodiments, the VH1/VL1 pair can bind to an effector cell protein, and the VH2/VL2 pair can bind to a target cell protein.

In one aspect, a bispecific antibody comprises one polypeptide chain or two different polypeptide chains having different amino acid sequences. The first polypeptide chain comprises an mFc domain with one or two amino acid substitutions and two VH regions plus two VL regions separated by linkers. The mFc domain can be N-terminal, C-terminal or in the middle relative to the four immunoglobulin variable regions, and it can be joined to the variable regions via a linker. The second polypeptide chain comprises an mFc domain.

Other kinds of alteration can also be part of an Fc polypeptide chain that is part of a Bi-mFc. In one aspect, an Fc region included in a Bi-mFc can comprise one or more "alterations that inhibit the binding of an Fc gamma receptor (FcyR)" to the Fc region as defined above. In another aspect, an Fc region included in a Bi-mFc can comprise one or more "Fc alterations that extend half-life," as defined above. In still another aspect, one or more "alterations that enhance ADCC" can be included in an Fc region that is part of a Bi-mFc.

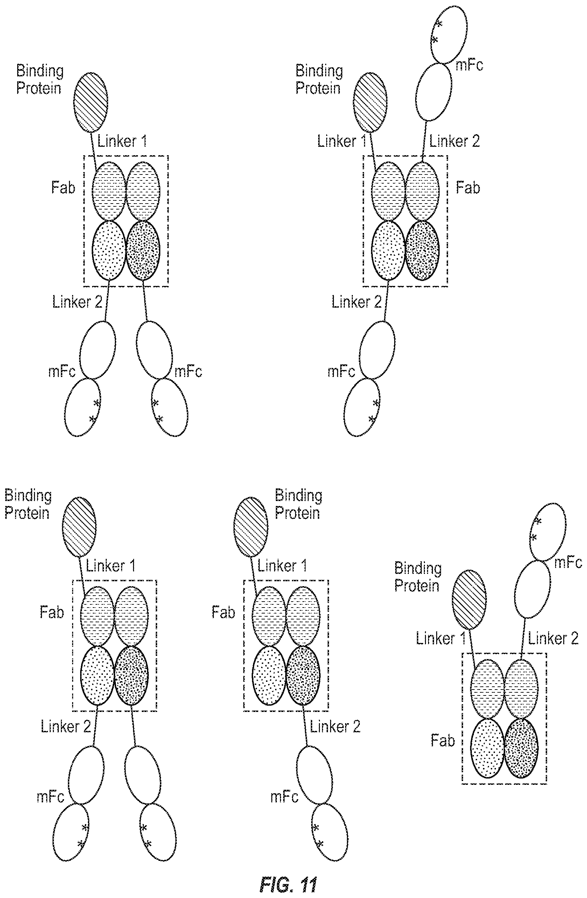

In some embodiments, an "i-shaped" bispecific antibodies (iBiBody) comprises two monomeric Fc (mFc) fusion proteins (FIG. 11). In certain embodiments, one of the monomeric Fc fusion proteins comprises a binding protein, an mFc domain, and a VL-CL domain. In one aspect, the said binding protein is connected to an N terminal or C terminal of VL-CL domain by a linker. The said mFc domain is linked to the opposite terminal of VL-CL domain by another linker. The other monomeric Fc fusion protein comprises an mFc domain and a VH-CH1 domain. In certain other embodiments, one of the monomeric Fc fusion proteins comprises a binding protein, an mFc domain and a VH-CH1 domain. In one aspect, the said binding protein is connected to an N terminal or C terminal of the VH-CH1 domain by a linker. The said mFc domain is linked to the opposite terminal of VH-CH1 by another linker. The other monomeric Fc fusion protein comprises an mFc domain and a VL-CL. The said VL, CL, VH and CH1 of two monomeric Fc fusion proteins form a Fab. In certain embodiments, the binding protein is an scFv. In certain embodiments, the linker connected to mFc may have one or more cysteine residues to form disulfide bonds.

In other embodiments, an "i-shaped" bispecific antibody (iBiBody) comprises one monomeric Fc(mFc) fusion protein and another polypeptide chain without mFc (FIG. 11). In one aspect, the said mFc and binding protein can be on one polypeptide chain of said iBiBody. In another aspect, the said mFc and binding protein can be on two different polypeptide chain of said iBiBody.

In certain embodiments, the monomeric Fc fusion proteins comprise a binding protein, an mFc domain, and a VL-CL domain.

In one aspect, the said binding protein is connected to an N terminal or C terminal of VL-CL domain by a linker.

The said mFc domain is linked to the opposite terminal of VL-CL domain by another linker. The other polypeptide chain comprises a VH-CH1 domain.

In certain other embodiments, the monomeric Fc fusion proteins comprise a binding protein, an mFc domain and a VH-CH1 domain.

In one aspect, the said binding protein is connected to an N terminal or C terminal of the VH-CH1 domain by a linker. The said mFc domain is linked to the opposite terminal of VH-CH1 by another linker. The other polypeptide chain comprises a VL-CL. The said VL, CL, VH and CH1 of said iBiBody form a Fab.

In further other embodiment, the monomeric Fc fusion protein comprises an mFc domain and a VH-CH1 domain. The said mFc domain is linked to N terminal or C terminal of the VH-CH1 domain by a linker. The other polypeptide chain comprises a VL-CL and a binding protein. The said binding protein is connected to N terminal or C terminal of VL-CL domain by a linker. The said VL, CL, VH and CH1 of said iBiBody form a Fab. In further other embodiment, the monomeric Fc fusion proteins comprise an mFc domain and a VL-CL domain. The said mFc domain is linked to N terminal or C terminal of VL-CL by a linker. The other polypeptide chain comprises a VH-CH1 and a binding protein. The said binding protein is linked to N terminal or C terminal of the VH-CH1 domain by a linker. The said VL, CL, VH and CH1 of said iBiBody form a Fab.

Other kinds of alteration can also be part of an Fc polypeptide chain that is part of an iBiBody. In one aspect, an mFc polypeptide included in an iBiBody can comprise one or more "linkers that inhibit the binding of an Fc gamma receptor (FcyR)" to the Fc region as defined above. In another aspect, an Fc region included in an iBiBody can comprise one or more "Fc alterations that extend half-life," as defined above. In still another aspect, one or more "alterations that enhance ADCC" can be included in an Fc region that is part of an iBiBody.

In one embodiment, an "i-shaped" bispecific antibody (iBiBody) comprising a CH3 domain with one or two amino acid substitutions can bind monovalently to two different antigens. In addition, it can bind to the neonatal Fc receptor (FcRn) at slightly acidic pH (about pH5.5-6.0) via its Fc region. This interaction with FcRn can lengthen the half-life of a molecule in vivo. An iBiBody can bind to an immune effector cell and a target cell and/or can mediate cytolysis of a target cell by an immune effector cell.

The redirected lysis of target cells via the recruitment of T cells by a multispecific, at least bispecific, antibody construct involves cytolytic synapse formation and delivery of perforin and granzymes. The engaged T cells are capable of serial target cell lysis, and are not affected by immune escape mechanisms interfering with peptide antigen processing and presentation, or clonal T cell differentiation; see, for example, WO 2007/042261.

Besides retargeting effector cells to the site of cancer, new applications were established for bispecific antibodies. Bispecific antibodies that can simultaneously bind to cell surface antigens and payloads are an ideal delivery system for therapeutic and diagnostic use. Bispecific antibodies that can inhibit two correlated signaling molecules at the same time can be developed to overcome inherent or acquired resistance and to be more efficient angiogenesis inhibitors. Bispecific antibodies can also be used to treat hemophilia A by mimicking the function of factor VIII. Bispecific antibodies also have broad application prospects in bone disorders and infections and diseases of the central nervous system.

### EXAMPLE

### Example 1

### Computational analysis for the identification of T366 and Y407 (Kabat numbering) at the human IgG1 Fc interface for mutagenesis

Computational analysis of a human IgG1 crystal structure (PDB entry 1HZH) revealed that the Y407 residue of CH3 (Kabat numbering scheme) plays a critical role in the homodimerization of Fc by creating a hydrophobic packing interaction. We also identified a void structure at the CH3 interface that is in close proximity to the Y407 residue and is lined by the T366 residue. We hypothesized that substitution of these two residues of CH3 could lead to new CH3 interfaces which allow for the stable formation of mFc. To test this hypothesis, we generated a phage-display library of Fc mutants by randomizing the T366 and Y407 residues with the degenerate codon NNS, which encodes the complete set of standard amino acids. The library was panned with protein A-conjugated resin to enrich clones with preserved binding to protein A, which indicates correct folding of the Fc mutants. Screening was followed with protein G-coated 96-well plates to identify individual clones which also bind to protein G (FIG. 1).

The atomic coordinates of Fc were extracted from the crystal structure of a human IgG1 antibody (Protein Data Bank entry 1HZH). All hydrophobic residues at the Fc interface were represented by using the PyMOL molecular graphics system (version 1.5.0.4; Schrödinger, LLC). Void structures at the interface were located by concomitantly visualizing the side chains of the amino acid residues at the interface. Single point mutations were modeled using the PyMOL mutagenesis wizard with an appropriate side-chain rotamer.

### Construction, panning and screening of a phage-display library of Fc mutants for identification of first-generation monomeric Fc (mFc)

Screening the library with protein G-coated 96-well plates by using soluble expression-based monoclonal ELISA (semELISA) (Chen et al., Mol Immunol 2010, 47:912) led to the identification of 7 mFc variants. They all have hydrophobic residues in position 407 of CH3. In contrast, there is no preferential use of types of amino acid residues in position 366.

The phage-display library of Fc mutants was constructed by site-directed random mutagenesis.
MFcF1, 5'-GATCGGCCCAGGCGGCCGCACCTGAACTCCTGGGG-3' (sense) (SEQ ID NO: 18);
MFcR1, 5'-CAGGCTGACCTGGTTCTTGG-3' (antisense) (SEQ ID NO: 19);
MFcF2, 5'-CAGGTCAGCCTGNNSTGCCTGGTCAAAGGCTTC-3' (sense) (SEQ ID NO: 20);
MFcR2, 5'-GAGGAAGAAGGAGCCGTC-3' (antisense) (SEQ ID NO: 21);
MFcF3, 5'-GGCTCCTTCTTCCTCNNSAGCAAGCTCACCGTGGAC-3' (sense) (SEQ ID NO: 22);
MFcR3, 5'-GATCGGCCGGCCTGGCCTTTACCCGGAGACAGGG-3' (antisense) (SEQ ID NO: 23).

To randomize the T366 and Y407 residues of Fc, three gene fragments, fragment 1 encoding the N terminal portion, fragment 2 encoding the middle portion (containing the T366 mutations), and fragment 3 encoding the C terminal portion (containing the Y407 mutations) of Fc, were amplified by PCR with Fc-encoding plasmid as a template and primer pairs MFcF1/MFcR1, MFcF2/MFcR2, and MFcF3/MFcR3, respectively. Fragments 2 and 3 were joined together by overlapping PCR with both templates in the same molarities for 7 cycles in the absence of primers and 15 additional cycles in the presence of primers MFcF2 and MFcR3. The product was then linked to fragment 1 by overlapping PCR with primers MFcF1 and MFcR3, resulting in the full-length fragment of Fc mutants. The final product was digested with SfiI and cloned into the phagemid vector pWC1. A phage library was prepared by electroporation of E. coli strain TG1 electroporation-competent cells (Lucigen) with desalted and concentrated ligation, as described previously (Chen et al., J Mol Biol 2008, 382: 779-789). To select Fc mutants with preserved binding to protein A, which indicates proper folding of Fc, we passed the phage library (1010 pfu) through a column containing 400 µl protein A agarose and washed the column with 10 mL PBS (pH7.4) twice. Bound phage was eluted by 0.1 mM acetic acid buffer (pH3.0) and neutralized by 1 M Tris-HCl buffer (pH9.0) at a volume 1/10 that of elution buffer. Log-phased TG1 cells were infected with the eluted phage and plated for single colonies. To identify individual Fc mutants that preserved binding to protein G, single colonies were randomly picked, inoculated into 96-well plates, and induced for protein expression with 1 mM isopropyl β-D-1-thiogalactopyranoside. After overnight incubation, the supernatants of individual clones were screened for binding to protein G by using soluble expression-based monoclonal ELISA (semELISA) as described previously (Chen et al., Mol Immunol 2010, 47: 912-921). Single colonies were randomly selected for sequencing, expression and characterization for monomeric states of purified mFc mutants. (FIG. 2).

Expression and purification of the seven mFc variants. The seven mFc variants were expressed in the E. coli strain HB2151 and purified from the soluble fraction of periplasm by using protein A-conjugated resin. On a non-reducing SDS-PAGE, the wild-type human IgG1 Fc migrated as a homodimer with apparent molecular weight (aMW) of approximately 50 kDa, while all the mFc variants ran as a monomer with aMW of approximately 30 kDa.(FIG. 3).

### Size-exclusion chromatography

A Superdex200 10/300 GL column (GE Healthcare) was calibrated with protein molecular mass standards of carbonic anhydrase (29 kDa), ovalbumin (44 kDa), conalbumin (75 kDa), aldolase (158 kDa) and ferritin (440 kDa). Purified proteins at a concentration of 0.5 mg mL-1 in PBS (pH7.4) were loaded onto the pre-equilibrated column and eluted with PBS (pH7.4) at 0.5 mL/min. Size-exclusion chromatography revealed that about 90% of the purified mFc7 protein in PBS (pH7.4) eluted as a monomer while other mFc variants formed more homodimers and higher-order oligomers. [FIG. 4][Table 1].

### Mutagenesis of the M407 residue of mFc7 for identification of second-generation mFc

We hypothesized that formation of Fc monomers could be further improved by refining the T366L and Y407M mutations in mFc7. 3 of 7 first-generation mFc variants contain the T366L mutation, suggesting that the mutation could be essential for the stable formation of Fc monomers. We therefore made a small phage-display library of mFc7 mutants by randomizing the M407 residue while leaving the T366L mutation intact. Direct screening of the library by using semELISA with protein G led to the identification of 9 second-generation mFc variants. In contrast to the first-generation variants which all have hydrophobic residues in position 407 of CH3, there is no preferential use of types of amino acid residues in position 407 of the second-generation variants. To randomize the M407 residue of mFc7, we used the following primers additionally:
mFc7F, 5'-GGCTCCTTCTTCCTCNNSAGCAAGCTCACCGTG-3' (sense) (SEQ ID NO: 24);
mFc7R: 5'-GAGGAAGAAGGAGCCGTC-3' (antisense) (SEQ ID NO: 25).

The gene fragments encoding the N and C terminal portions of mFc7 were PCR amplified by using mFc7-encoding plasmid as a template and primer pairs MFcF1/mFc7R and mFc7F/MFcR3, respectively. The full-length gene fragment of mFc7 mutants was assembled by overlapping PCR with the primary PCR products as templates and primers MFcF1 and MFcR3. The final product was digested with SfiI and cloned into the phagemid vector pWC1. DH5α chemical competent cells (Lucigen) were transformed and plated on agar plates. Single colonies were randomly selected for sequencing, expression and characterization for monomeric states of purified mFc7 mutants. [FIG. 5]

Fc variants were purified from the soluble fraction of HB2151 periplasm by Protein A Sepharose 4 Fast Flow column chromatography (GE Healthcare) according to the manufacturer's protocols. Fc protein homogeneity analysis (SEC) was performed using a Superdex200 10/300 GL column (GE Healthcare). Protein concentrations were determined by measuring the absorption at 280 nm and calculation using 1 mg/ml=1.74 OD280.

Size-exclusion chromatography (SEC) revealed that no less than 80% of the purified mFc7.21, mFc7.22, mFc7.24, mFc7.27, and mFc7.28 proteins in PBS (pH7.4) eluted as a monomer. mFc7.21, mFc7.24 and mFc7.28 showed a higher percentage of monomers than mFc7 while no improvement was observed with mFc7.22 (FIG. 6) [Table 1]. This invention provides, for the first time, that mFc can be successfully generated with only two amino acid mutations, which could lead to no or less immunogenicity in humans compared to previously reported mFc.

**Table 1. Example of oligomeric status of mFc variants measured by SEC.**

| **mFc variants** | **Monomer (%)** | **Dimer (%)** | **Oligomer (%)** |
|---|---|---|---|
| mFc3 **(SEQ ID NO: 3)** | 60 | 40 | 0 |
| mFc7 **(SEQ ID NO: 5)** | 90 | 10 | 0 |
| mFc8 **(SEQ ID NO: 6)** | 58 | 38 | 4 |
| mFc12 **(SEQ ID NO: 7)** | 45 | 55 | 0 |
| mFc7.21(**SEQ ID NO: 13)** | 97 | 3 | 0 |
| mFc7.22(**SEQ ID NO: 14)** | 80 | 20 | 0 |
| mFc7.24(**SEQ ID NO: 15)** | 95 | 5 | 0 |
| mFc7.27(**SEQ ID NO: 16)** | 90 | 10 | 0 |
| mFc7.28(**SEQ ID NO: 17)** | 96 | 4 | 0 |

### Example 2 Bispecific antibody

### Cloning of a proof-of-concept bispecific antibody with CH1-CK for heterodimerization and mFc7.x for extended half-life.

We propose that mFc could be used to generate a novel bispecific antibody format which could successfully address the potential issues with some well-known bispecific antibody formats such as the bispecific T cell engager (BiTE) (Amgen) and CrossMab (Roche). In our newly proposed format, human IgG CH1 and CL (kappa or lambda) are used as a scaffold for efficient heterodimerization and mFc is used to avoid the issue with Fc homodimerization and extend the half-life of bispecific antibodies in vivo. Specifically, in one arm of our bispecific antibodies, an scFv or other binding proteins is fused via a polypeptide linker to the N terminus of CL; the latter is further fused to the N terminus of mFc through the same or a different polypeptide linker. In the other arm of our bispecific antibodies, an scFv or other binding proteins is fused via a polypeptide linker to the N terminus of CH1; the latter is further fused to the N terminus of mFc through the same or a different polypeptide linker. Examples of the polypeptide linkers include but are not limited to the G4S repeats and full-length or partial IgG hinge sequences.

### Generation of bispecific antibodies

To provide a proof-of-concept, we randomly selected two scFvs (scFv 1 and scFv 2) to generate a bispecific antibody in the proposed format. We used a previously constructed bispecific antibody with CH1-CK, designated BiCD20CD3 (unpublished), as a model to generate a novel bispecific antibody with mFc7.x for extended half-life.

The following primers were used:
bnIgG20L1, 5'-GTGTAAGCTTACCATGGGTGTGCCCACTCAGGTCCTGGGGTTGCTG-3' (sense) (SEQ ID NO: 26);
LCKR, 5'-TGGGCACGGTGGACACTCTCCCCTGTTGAAGC-3' (antisense) (SEQ ID NO: 27);
MFc7.xF1, 5'-TGTCCACCGTGCCCAGCACCTGAACTCCTGGGG-3' (sense); (SEQ ID NO: 28)
MFc7.xR1, 5'-GATCGAATTCTTATTTACCCGGAGACAGGG-3' (antisense); (SEQ ID NO: 29)
bnIgG20H1, 5'-GTGTTCTAGAGCCGCCACCATGGAATGGAGCTGGGTCTTTCTCTTC-3' (sense) (SEQ ID NO: 30);
HCHR, 5'-TGGGCACGGTGGACAAGATTTGGGCTCAAC-3' (antisense) (SEQ ID NO: 31);
MFc7.xR2, 5'-CGGCCGTCGCACTCATTTACCCGGAGACAGGG-3' (antisense); (SEQ ID NO: 32)
AAF, 5'-TGAGTGCGACGGCCGGCA-3' (sense) (SEQ ID NO: 33);
AAAR, 5' -CCCGAGGTCGACGCTCTC-3' (antisense) (SEQ ID NO: 34).

In BiCD20CD3, an anti-CD20 scFv (designated scFv 1 hereafter) was fused to the N terminus of CK via the G4S polypeptide linker while an anti-CD3 scFv (designated scFv 2 hereafter) was linked to the N terminus of CH1 via the same linker. To clone the new bispecific antibody with mFc7.x, scFv1-CK and scFv 2-CH1 gene fragments were PCR amplified with BiCD20CD3 plasmid as a template and primer pairs bnIgG20L1/LCKR and bnIgG20H1/HCHR, respectively. Two mFc7.x gene fragments were PCR amplified with mFc7.x plasmid as a template and primer pairs MFc7.xF1/MFc7.xR1 and MFc7.xF1/MFc7.xR2, respectively. ScFv 1-CK and scFv 2-CH1 were joined to the two mFc7.x fragments by overlapping PCR with primer pairs bnIgG20L1/MFc7.xR1 and bnIgG20H1/MFc7.xR2, respectively. scFv 2-CH1-mFc7.x was further linked to a poly A signal gene fragment (polyA), which was PCR amplified with pDin1 as a template and primers AAAF and AAAR, by overlapping PCR with primers bnIgG20H1 and AAAR. ScFv 1-CK-mFc7.x and scFv 2-CH1-mFc7.x-polyA were sequentially cloned into the pDin1 vector via the HindIII/EcoRI and XbaI/SalI restriction sites, respectively.

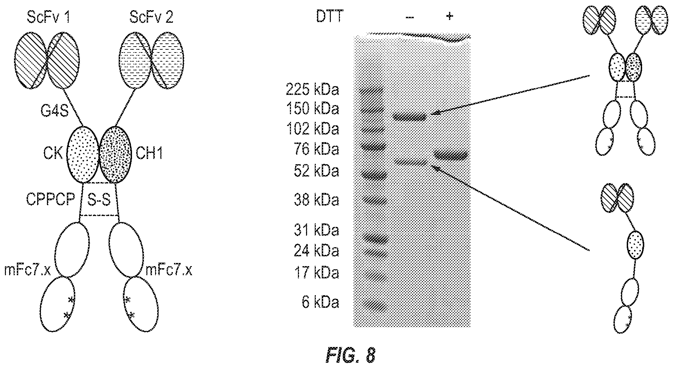

As in FIG. 8, human antibody kappa light chain constant region (CK) and human IgG1 constant region 1 (CH1) were used for heterodimerization and mFc7.x was used to extend the half-life of the bispecific antibody. Specifically, in one arm, scFv 1 was fused to the N terminus of CK via a polypeptide linker composed of a single copy of the G4S motif and CK was further fused to the N terminus of mFc7.x via a polypeptide linker composed of five amino acid residues CPPCP, part of the human IgG1 hinge sequence. In the other arm, scFv 2 was fused to the N terminus of CH1 and CH1 was further fused to the N terminus of mFc7.x via the same linkers, respectively. The G4S linkers provide sufficient flexibility for scFvs to exert their functions while the CPPCP linkers create two inter-chain disulfide bonds to stabilize the heterodimer. The bispecific antibody was cloned into Dinova expression vector pDin1, which allows simultaneous expression of two different gene cassettes. It was transiently expressed in 293 free style cells and affinity purified from the culture supernatant by using GE Healthcare HiTrap KappaSelect resin according to the manufacturer's instructions. On a non-reducing SDS-PAGE, the majority of purified protein migrated as an expected heterodimer with apparent molecular weight (aMW) of approximately 120 kDa, which is close to its calculated molecular weight (cMW) of 124 kDa. The rest of the protein was the dissociated scFv 1-CK-mFc7.x chain with aMW of approximately 60 kDa, which is also close to its cMW of 62 kDa. Under reducing condition, the dissociated scFv 1-CK-mFc7.x and scFv 2-CH1-mFc7.x chains were not well separated due to almost the same cMW of 62 kDa (FIG. 8).

### Size-exclusion chromatography (SEC) of the purified bispecific antibody.

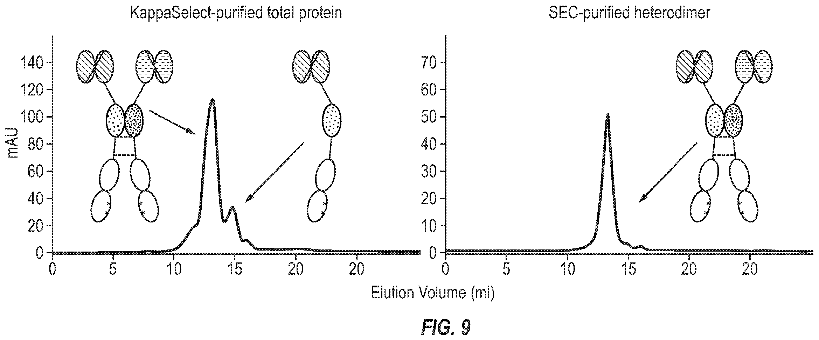

SEC revealed that the KappaSelect-purified bispecific antibody contained dissociated scFv 1-CK-mFc7.x monomer, heterodimer and higher-order oligomers which could be well separated from each other by SEC leading to a relatively pure heterodimer of scFv 1-CK-mFc7.x/scFv 2-CH1-mFc7.x. A Superdex200 10/300 GL column (GE Healthcare) was calibrated with protein molecular mass standards of carbonic anhydrase (29 kDa), ovalbumin (44 kDa), conalbumin (75 kDa), aldolase (158 kDa) and ferritin (440 kDa). Purified proteins at a concentration of 0.5 mg mL-1 in PBS (pH7.4) were loaded onto the pre-equilibrated column and eluted with PBS (pH7.4) at 0.5 mL/min. (FIG. 9).

### Example 3. Bispecific mFc antibodies with only one polypeptide chain.

Examples of bispecific mFc antibodies with only one polypeptide chain are shown in FIG. 10. In one format, scFv 1 and scFv 2 are fused to the N and C terminus, respectively, of mFc via a polypeptide linker (a). In another format, scFv 1 is fused to the N terminus of scFv 2 via a polypeptide linker and the latter is further linked to the N terminus of mFc via the same or a different polypeptide linker (b). Yet in another format, mFc is fused to the N terminus of scFv 1 and the latter is further linked to the N terminus of scFv 2 via the same or a different polypeptide linker (c). scFv 1 or scFv 2 could also be other binding proteins.

### Example 4. "i-Shaped" Bispecific antibody (iBiBody)

The design of a novel "i-shaped" bispecific antibody format (iBiBody) with VH-CH1 and VL-CL of a Fab for heterodimerization and mFc for extended half-life. mFc could be used to generate a novel bispecific antibody format which could successfully address the potential issues with some well-known bispecific antibody formats such as the bispecific T cell engager (BiTE) (Amgen) and CrossMab (Roche). A novel "i-shaped" asymmetric bispecific antibody format (iBiBody) is designed comprising a binding protein, a Fab and one or more monomeric Fc polypeptide, wherein the said binding protein is linked to an N terminal or C-terminal of said Fab and wherein the said one or more monomeric Fc polypeptides are linked to the other terminals of said Fab. Examples of iBiBody design are shown in FIG. 11. Examples of the polypeptide linkers include but are not limited to the G4S repeats and full-length or partial IgG hinge sequences.

It is hypothesized that the use of a shortened human IgG1 hinge sequence such as CPPCP in between Fab and mFc could lead to decreased binding to Fc receptors (FcR) by creating a steric hindrance, which diminishes FcR binding-mediated toxicity such as lymphopenia, and increased antibody stability by removing protease cleavage sites.

## Claims

1. A monomeric Fc polypeptide comprising a sequence selected from the group consisting of SEQ ID NO: 9, 13, 15, and 17.

2. A monomeric Fc fusion protein comprising the monomeric Fc polypeptide of claim 1 and an antibody variable domain.

3. A polynucleotide encoding the monomeric Fc polypeptide of claim 1.

4. An expression vector comprising a polynucleotide of claim 3 encoding the monomeric Fc polypeptide.

5. A host cell comprising an expression vector of claim 4 encoding the monomeric Fc polypeptide.

6. A method of preparing a monomeric Fc polypeptide, wherein said method comprises the steps of: (a) culturing host cells comprising a DNA encoding the monomeric Fc polypeptide of claim 1 or the monomeric Fc fusion protein of claim 2 under conditions wherein said monomeric Fc polypeptide or said monomeric Fc fusion protein is expressed; and (b) recovering the monomeric Fc polypeptide or the monomeric Fc fusion protein from the host cell culture.

7. A bispecific antibody comprising a monomeric Fc polypeptide of claim 1 or the monomeric Fc fusion protein of claim 2.

## Patentansprüche

1. Ein monomeres Fc-Polypeptid umfassend eine Sequenz ausgewählt aus der Gruppe bestehend aus SEQ ID NO: 9, 13, 15 und 17.

2. Ein monomeres Fc-Fusionsprotein umfassend das monomere Fc-Polypeptid aus Anspruch 1 und eine variable Antikörperdomäne.

3. Ein Polynukleotid, das das monomere Fc-Polypeptid aus Anspruch 1 kodiert.

4. Ein Expressionsvektor umfassend ein Polynukleotid aus Anspruch 3, das das monomere Fc-Polypeptid kodiert.

5. Eine Wirtszelle umfassend einen Expressionsvektor aus Anspruch 4, der das monomere Fc-Polypeptid kodiert.

6. Ein Verfahren zur Herstellung eines monomeren Fc-Polypeptids, wobei jenes Verfahren folgende Schritte umfasst: (a) Kultivieren von Wirtszellen, die eine DNA umfassen, die das monomere Fc-Polypeptid aus Anspruch 1 oder das monomere Fc-Fusionsprotein aus Anspruch 2 kodiert, unter Bedingungen, wobei jenes monomere Fc-Polypeptid oder jenes monomere Fc-Fusionsprotein exprimiert wird; und (b) Gewinnen des monomeren Fc-Polypeptids oder des monomeren Fc-Fusionsproteins aus der Wirtszellkultur.

7. Ein bispezifischer Antikörper umfassend ein monomeres Fc-Polypeptid aus Anspruch 1 oder das monomere Fc-Fusionsprotein aus Anspruch 2.

## Revendications

1. Polypeptide Fc monomère comprenant une séquence sélectionnée dans le groupe consistant en SEQ ID NO : 9, 13, 15 et 17.

2. Protéine de fusion de Fc monomère comprenant le polypeptide Fc monomère selon la revendication 1 et un domaine variable d'anticorps.

3. Polynucléotide codant pour le polypeptide Fc monomère selon la revendication 1.

4. Vecteur d'expression comprenant un polynucléotide selon la revendication 3 codant pour le polypeptide Fc monomère.

5. Cellule hôte comprenant un vecteur d'expression selon la revendication 4 codant pour le polypeptide Fc monomère.

6. Procédé de préparation d'un polypeptide Fc monomère, dans lequel ledit procédé comprend les étapes suivantes : (a) la mise en culture de cellules hôtes comprenant un ADN codant pour le polypeptide Fc monomère selon la revendication 1 ou la protéine de fusion de Fc monomère selon la revendication 2 dans des conditions dans lesquelles ledit polypeptide Fc monomère ou ladite protéine de fusion de Fc monomère est exprimé ; et (b) la récupération du polypeptide Fc monomère ou de la protéine de fusion de Fc monomère à partir de la culture de cellules hôtes.

7. Anticorps bispécifique comprenant un polypeptide Fc monomère selon la revendication 1 ou la protéine de fusion de Fc monomère selon la revendication 2.
